# EUROPEAN PATENT SPECIFICATION

(11) **EP 4 110 442 B1**
(45) Date of publication and mention of the grant of the patent: **11.02.2026**
(21) Application number: 21715992.0
(22) Date of filing: 24.02.2021
(51) Int. Cl.: A61M 15/00, B05D 5/08, B05D 7/00, A61K 9/00, A61K 31/137

(54) **INHALER**
INHALATOR
INHALATEUR

(30) Priority: 28.02.2020 US 202062982823 P
(43) Date of publication of application: 04.01.2023
(73) Proprietor: Kindeva Drug Delivery L.P., Woodbury, MN 55129 (US)
(72) Inventor: COCKS, Philip M., St. Paul, Minnesota 55170 (US)
(74) Representative: Vossius & Partner Patentanwälte Rechtsanwälte mbB
(86) International application number: PCT/US2021/019359
(87) International publication number: WO 2021/173627

(56) References cited:
- WO-A1-2016/164508
- WO-A1-2019/236649
- US-A1- 2017 152 396

## Description

### BACKGROUND

An inhaler for delivering albuterol (also known in the art as salbutamol) is available from VENTOLIN under the trade designation EVOHALER (GlaxoSmithKline plc, Brentford, UK). The EVOHALER inhaler provides about 100 micrograms (µg) of albuterol as albuterol sulfate per actuation, using a valve with a 63 µL volume and a propellant, particularly a hydrofluorocarbon (HFC) propellant. The volume of the valve provides the volume of material that is released from the inhaler during each actuation. Each EVOHALER inhaler delivers 200 doses of albuterol.

US Patent Publication Number 2017/0152396, teaches coatings for inhaler components

Patent documents WO2019/236649A1 and WO2016/164508A1 are hereby acknowledged.

### DETAILED DESCRIPTION

Throughout this disclosure, singular forms such as "a," "an," and "the" are often used for convenience; however, the singular forms are meant to include the plural unless the singular alone is explicitly specified or is clearly indicated by the context. When the singular alone is called for, the term "one and only one" is typically used.

Some terms in this disclosure are defined below. Other terms will be familiar to the person of skill in the art and should be afforded the meaning that a person of ordinary skill in the art would have ascribed to them.

Terms indicating a high frequency, such as (but not limited to) "common," "typical," and "usual," as well as "commonly," "typically," and "usually" are used herein to refer to features that are often employed in the invention and, unless specifically used with reference to the prior art, are not intended to mean that the features are present in the prior art, much less that those features are common, usual, or typical in the prior art.

The term "independently" when used in reference to variables means that the identity of one variable bears no relation to the identity of another. For example, if each variable X in item X-X is independently chosen from A and B, then X-X may be A-A, A-B, B-A, or B-B.

Except where noted, in this disclosure, the mass and concentrations (when expressed in terms that depend on mass) of albuterol, its salts, and their hydrates, are expressed in terms of free albuterol. When albuterol sulfate anhydrate, which is the most common form of albuterol used in pharmacy, or a different salt or hydrate of albuterol is employed, the quantity of the salt or hydrate of albuterol can be calculated by comparing the molecular weight of the substance employed to the molecular weight of free albuterol and making an appropriate calculation, as known to the skilled artisan, to determine the quantity to be used. When the mass or concentration of albuterol is expressed in terms of another form, such as a salt or hydrate, this is noted with a statement that the mass or concentration is expressed as the other form of albuterol.

This disclosure recognizes several technical problems with albuterol containing inhalers. One problem is that known inhalers release a relatively large amount of propellant, usually a hydrofluorocarbon such as HFC-227 or HFC-134a, which may be detrimental to the environment, and/or contribute to global warming. This problem may also be formulated as how to reduce the volume of propellant released from the inhaler in each actuation. Another problem is that albuterol is a tacky substance that, if present in too high a concentration in an inhaler canister, tends to stick to the valve, particularly to the valve step, which in turn results in unacceptable dose to dose variation in the amount of albuterol released, damage to the valve, or both. This problem may also be formulated as how to prevent albuterol from sticking to the components of the inhaler, particularly the valve and most particularly the valve stem, in higher concentrations. Yet another problem is that the cost and environmental impact of shipping medicaments, such as inhalers, is increasingly high as more medicaments become available. This problem may also be formulated as to how to produce an inhaler with a reduced mass, particularly with a reduced mass of the composition that contains the albuterol, while retaining the same number of doses per inhaler; reducing this mass permits smaller inhaler components, such as a smaller canister, to be used, which in turn can reduce the overall mass of the inhaler by more than the reduction in mass of the albuterol composition.

Briefly, a solution that may be applied to these and other problems lies in an inhaler comprising a valve having a valve volume of no more than 40 microliters (µL). At least a portion of the valve is coated with a valve coating. The valve coating comprising the condensation product of a first layer on the at least a portion of the valve and a second layer on the first layer. The inhaler also comprises a pressurized canister in fluid communication with the valve. Within the pressurized canister is a pharmaceutically acceptable inhalable composition that comprises a propellant and albuterol, one or more pharmaceutically acceptable salts thereof, or one or more pharmaceutically acceptable hydrates of any of the foregoing. The albuterol, one or more pharmaceutically acceptable salts thereof, or one or more pharmaceutically acceptable hydrates of any of the foregoing is present in the pressurized canister in a concentration of 2 milligrams/milliliter (mg/mL) or greater.

In principle, any commercially available inhaler valve having the requisite volume can be used. Examples include valves available from APTARGROUP (Crystal Lake, IL, USA) and Consort Medical (Hertfordshire, UK). Particular valves that can be used include those with valve volumes no more than 35 µL, more particularly no more than 30 µL, even more particularly no more than 27.5 µL, or most particularly about 25 µL. Particularly, the valve can be a metered valve. In such cases, the inhaler will typically be a metered dose inhaler.

The valve coating is designed to prevent albuterol from sticking to the valve and thereby reduce or eliminate unacceptable dose to dose variation as well as damage to the valve. The valve coating is disposed on at least a portion of the valve. Particularly, the valve comprises a valve step and the valve coating is disposed on at least a portion of the valve stem; the valve coating may also be disposed on other parts of the valve or on other components of the inhaler, such as the interior of the pressurized canister.
The valve coating is the condensation product of a two-layer coating. The first layer is disposed directly or indirectly on at least a portion of the inhaler. The second layer is disposed on the first layer.

The first layer is typically disposed directly on the at least a portion of the valve, though it may also be disposed indirectly thereon, and contains a silane with reactive silane groups. The second layer comprises an at least partially fluorinated compound having at least one reactive silane groups, most typically one or more of hydrolysable silane groups or hydroxysilane groups. The second layer is deposited on the first layer, such that the reactive silane groups of the first layer can undergo a chemical reaction, such as a condensation reaction, with the reactive silane groups of the second layer.

With regard to the first layer, the at least one reactive silane group may be of formula Si(R⁰)ₙX₃₋ₙ, wherein R⁰ is a substantially non-hydrolysable group, X is a hydrolysable or hydroxy group and n is 0, 1 or 2.

More commonly the first layer typically comprises a silane having two or more reactive silane groups separated by an organic linker. In particular cases the silane having two or more reactive silane groups is of Formula (I)

X₃₋ₘ (R¹)ₘSi - Q - Si(R²)ₖ X₃₋ₖ (I)

wherein R¹ and R² are independently selected univalent groups such as C1-C4 alkyl, X is a hydrolysable or hydroxy group, m and k are independently 0, 1, or 2 and Q is a divalent organic linking group.

Usually, Q will comprise a 1 to 12 atom chain, more usually a substituted or unsubstituted C₂ to C₁₂ hydrocarbyl chain. Preferably, Q comprises a substituted or unsubstituted C₂ to C₁₂ alkyl chain.

Useful examples of silanes having two or more reactive silane groups include one or a mixture of two or more of 1,2- bis(trialkoxysilyl) ethane, 1,6- bis(trialkoxysilyl) hexane, 1,8- bis(trialkoxysilyl) octane, 1,4-bis(trialkoxysilylethyl)benzene, bis(trialkoxysilyl)itaconate, and 4,4'-bis(trialkoxysilyl)-1,1'-diphenyl, wherein any trialkoxy group may be independently trimethoxy or triethoxy.

Q may comprise a chain substituted by one or more atoms of N, O, and/or S. Thus, further examples of the silane having two or more reactive silane groups include one or a mixture of two or more of bis(trialkoxysilylpropyl)amine; bis (3-trialkoxysilylpropyl) ethylenediamine; bis (3-trialkoxysilylpropyl) n-methylamine; bis[3-(trialkoxysilyl)propyl]fumarate and N, N-bis (3-trialkoxysilylmethyl) allylamine, wherein any trialkoxy group may be independently trimethoxy or triethoxy for example.

In particular embodiments, the silane is such that Q may be of formula - (CH₂)ᵢ-A-(CH₂)ⱼ - wherein A is NRⁿ, O, or S; i and j are independently 0, 1, 2, 3 or 4 and wherein Rⁿ is H or C₁ to C₄ alkyl. Even more preferably, Q may be of formula - (CH₂)ᵢ -NH - (CH₂)ⱼ - and i and j are each independently 1, 2, 3 or 4. Most preferably i and j are each 3.

A particular second layer can be a perfluoropolyether silane according to Formula Ia in which R^{f} comprises from 20 to 40 linked repeating units confer additional lubricity compared to those with fewer repeating units, and when these are assembled with other components to make up valves, the valves have lower actuation forces.

Formula Ia is:

R*^{f}*[Q¹-[C(R)₂-Si(Y)₃₋ₓ(R^{1a})ₓ]_{y}]_{z} Ia

wherein:
R*^{f}* is a monovalent or multivalent polyfluoropolyether moiety;
Q¹ is an organic divalent or trivalent linking group;
each R is independently hydrogen or a C1-4 alkyl group;
each Y is independently a hydrolysable group;
R^{1a} is a C1-8 alkyl or phenyl group;
x is 0 or 1 or 2;
y is 1 or 2; and
z is 1, 2, 3, or 4.

In some particular cases of Formula Ia, x, y, and z are each 1.

In some particular cases of Formula Ia, Y is O-

A more particular second layer is a perfluoropolyether silane of Formula Ib.

R*^{f}*[Q¹-[C(R)₂-Si(O-)₃₋ₓ(R^{1a})ₓ]_{y}]_{z} Ib

wherein:
R*^{f}* is a monovalent or multivalent polyfluoropolyether segment;
Q¹ is an organic divalent or trivalent linking group;
each R is independently hydrogen or a C1-4 alkyl group;
R^{1a} is a C1-8 alkyl or phenyl group;
x is 0 or 1 or 2;
y is 1 or 2; and
z is 1, 2, 3, or 4.

The second layer may comprise a polyfluoroether silane, in particular a polyfluoropolyether silane. More particularly, the polyfluoroether silane may be a perfluorinated polyfluoroether moiety, even more particularly the polyfluoroether silane may be a perfluorinated polyfluoropolyether silane.

The polyfluoropolyether silane may be of formula (Ic)

R*^{f}*Q¹ᵥ[Q²_{w}-[C(R⁴)₂-Si(X)₃₋ₓ(R⁵)ₓ]_{y}]_{z} (Ic)

wherein:
R*^{f}* is a polyfluoropolyether moiety;
Q¹ is a trivalent linking group;
each Q² is an independently selected organic divalent or trivalent linking group;
each R⁴ is independently hydrogen or a C₁₋₄ alkyl group;
each X is independently a hydrolysable or hydroxyl group;
R⁵ is a C₁₋₈ alkyl or phenyl group;
v and w are independently 0 or 1, x is 0 or 1 or 2; y is 1 or 2; and z is 2, 3, or 4.

The polyfluoropolyether moiety R*^{f}* may comprise perfluorinated repeating units selected from the group consisting of -(CₙF₂ₙO)-,-(CF(Z)O)-, -(CF(Z)CₙF₂ₙO)-, -(CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6. In particular, n may be an integer from 1 to 4, more particularly from 1 to 3. For repeating units including Z the number of carbon atoms in sequence may be at most four, more particularly at most 3. Usually, n is 1 or 2 and Z is an -CF₃ group, more wherein z is 2, and R*^{f}* is selected from the group consisting of -CF₂O(CF₂O)ₘ(C₂F₄O)ₚCF₂-, -CF(CF₃)O(CF(CF₃)CF₂O)ₚCF(CF₃)-, -CF₂O(C₂F₄O)ₚ CF₂-, -(CF₂)₃O(C₄F₈O)ₚ(CF₂)₃-, -CF(CF₃)-(OCF₂CF(CF ₃))ₚO-CₜF₂ₜ-O(CF(CF₃)CF₂ O)ₚCF(CF₃)-, wherein t is 2, 3 or 4, m is 1 to 50, and p is 3 to 40.

The first layer or second layer can be applied, for example to the interior of the canister or the valve, by any suitable method. Independently of the application step used for the second layer, the first layer can be applied by spraying, dipping, rolling, brushing, spreading or flow coating, in particular for example by spraying or dipping. Independently of the application step used for the first layer, the second layer can be applied by spraying, dipping, rolling, brushing, spreading or flow coating, in particular for example by spraying or dipping.

In any of the above-mentioned ways of applying the first layer or the second layer, a coating liquid is usually used. Typically, the coating liquid comprises an alcohol or a hydrofluoroether.

If the coating liquid is an alcohol, preferred alcohols are C₁ to C₄ alcohols, in particular, an alcohol selected from ethanol, n-propanol, or iso-propanol or a mixture of two or more of these alcohols.

If the coating liquid is an hydrofluoroether, it is preferred if the coating solvent comprises a C₄ to C₁₀ hydrofluoroether. Generally, the hydrofluoroether will be of formula II

C_{g}F_{2g+1}OCₕH₂ₕ₊₁ (II)

wherein g is 2, 3, 4, 5, or 6 and h is 1, 2, 3 or 4. Examples of suitable hydrofluoroethers include those selected from the group consisting of methyl heptafluoropropylether, ethyl heptafluoropropylether, methyl nonafluorobutylether, ethyl nonafluorobutylether and mixtures thereof.

A cross-linking agent can be applied along with the first layer, the second layer, or both the first and second layers. The cross-linking agent may comprise a compound selected from group consisting of tetramethoxysilane; tetraethoxysilane; tetrapropoxysilane; tetrabutoxysilane; methyl triethoxysilane; dimethyldiethoxysilane; octadecyltriethoxysilane; 3-glycidoxy-propyltrimethoxysilane; 3-glycidoxy-propyltriethoxysilane; 3-aminopropyl-trimethoxysilane; 3-aminopropyl-triethoxysilane; bis ( 3-trimethoxysilylpropyl) amine; 3-aminopropyl tri(methoxyethoxyethoxy) silane; N ( 2-aminoethyl)3-aminopropyltrimethoxysilane; bis ( 3-trimethoxysilylpropyl) ethylenediamine; 3-mercaptopropyltrimethoxysilane; 3-mercaptopropyltriethoxysilane; 3-trimethoxysilyl-propylmethacrylate; 3-triethoxysilypropylmethacrylate; bis ( trimethoxysilyl ) itaconate; allyltriethoxysilane; allyltrimethoxysilane; 3-(N-allylamino)propyltrimethoxysilane; vinyltrimethoxysilane; vinyltriethoxysilane; and mixtures thereof.

Before applying the first layer, a pre-treatment step may be employed. The pre-treatment step will typically comprise cleaning the surface with a cleaning liquid. A particularly useful cleaning liquid comprises a hydrofluoroether, such as HFE72DE, an azeotropic mixture of about 70%w/w trans-dichloroethylene; 30% w/w of a mixture of methyl and ethyl nonafluorobutyl and nonafluoroisobutyl ethers.

After application of the first layer, it may be desirable to cure the first layer. Curing can be affected by evaporating the coating liquid in the presence of humidity, because water can promote curing of the first layer. Heat can also aid in promoting curing of the first layer. When heat is applied, the temperature should be low enough as to not deform the valve or canister, which are often (though not always) made of plastics.

Example of coatings that can be used as valve coatings can be found in US Patent Publication Number 2017/0152396, particularly for example in Example 2 of that publication.

Albuterol, a salt thereof, or a hydrate of any of the foregoing, and a propellant may be present within the pressurized canister. Other drugs or excipients may also be present.

The albuterol, salt thereof, or a hydrate of any of the foregoing may be present in an concentration of 2 mg/mL or greater, 2.5 mg/mL or greater, optionally 3 mg/mL or greater, further optionally 3.5 mg/mL or greater, still further optionally 3.75 mg/mL or greater, yet further optionally 4 mg/mL or greater, or even further optionally 4.5 mg/mL or greater.

The pressurized canister also contains a propellant. Most commonly, HFC propellants (sometimes known as HFA propellants) are employed. Particularly, HFC-134a or HFC-227 may be used, most particularly HFA-134a may be used.

Other materials, particularly excipients, may be present within the pressurized canister if needed, so long as the other materials are suitable for use in an inhaled formulation. Examples include surfactants, such as polyethylene glycol (PEG), and solubilizers, such as ethanol.

The inhaler may have other components depending on the desired use and function. In particular, the inhaler may also include a dose counter, such as those known in the art.

Typically, the inhaler also includes an actuator. When actuated, the actuator releases the contents of the pressurized canister from the inhaler. Upon one actuation of the actuator, the actuator typically releases about 70 µg to about 140 µg, particularly about 80 µg to about 125 µg, more particularly about 90 µg to about 110 µg, still more particularly about 95 µg to 105 µg, or yet still more particularly about 100 µg of albuterol from the inhaler. In this disclosure, the amount of albuterol "released" from the inhaler is the amount that can be collected outside the inhaler, and can be determined, for example, by collecting all of the material that is expelled from the inhaler and then determining the weight of the albuterol collected (by drying and weighing the albuterol, by using chromatography, such as high performance liquid chromatography (HPLC), or other means known in the art.) The precise amount of albuterol released from the inhaler will depend on the valve volume and the formulation concentration of the albuterol in the pressurized canister, and so can be selected by the person or ordinary skill in the art depending on the intended use and required dosing.

In use, an inhaler as described is actuated or otherwise manipulated to release the albuterol, one or more pharmaceutically acceptable salts thereof, or one or more pharmaceutically acceptable hydrates thereof from the inhaler. The albuterol, one or more pharmaceutically acceptable salts thereof, or one or more pharmaceutically acceptable hydrates of any of the foregoing is typically released through the valve, along with propellant and any other contents of the pressurized canister.

The method of use can be a method of treating a subject. In such methods, the albuterol, one or more pharmaceutically acceptable salts thereof, or one or more pharmaceutically acceptable hydrates of any of the foregoing is released into the airway of the subject.

### Examples

HFA-134a (1,1,1,2-tetrafluoroethane) was obtained from Daiken Industries Ltd. (Osaka, Osaka Prefecture, Japan). Albuterol sulfate, labeled salbutamol sulfate, (micronized with reported Certificate of Analysis: d100 of not more than (NMT) 10 micrometers, d92 of NMT 5 micrometers, and less than 1 micrometer of NMT 25%) was obtained from Sicor Societa' Italina Corticosteroidi Srl, Milan, Italy.

### Example 1

Each metered dose inhaler (MDI) was prepared using a 14 mL aluminum canister with the internal surface coated using a fluorocarbon polymerization (FCP) plasma treatment process (obtained from H&T Presspart, Blackburn, UK), a 25 microliter 3M retention type valve with a PBT (polybutylene terephthalate) stem and an EPDM (ethylene-propylene diene terpolymer elastomer) diaphragm seal (3M Corporation, Maplewood, MN), and a 3M Mk6S actuator having a 0.5 mm exit orifice diameter and 1.5 mm jet length (3M Corporation, Maplewood, MN). The bottle emptier, tank, spring, and ferrule components of the valves were coated with a fluoropolymer coating according to the general process described in Example 2 of U.S. Patent Application Publication Number 2017/0152396. The canisters were cold filled with 9.5 grams (g) of a suspension of albuterol sulfate (4.8026 mg/mL) in HFA-134a. The bulk suspension formulation for cold filling individual canisters was prepared by combining albuterol sulfate and the HFA-134a propellant in a vessel chilled to less than -40 °C. The suspension was mixed at 7000 rpm for 2 minutes using a Silverson mixer (Silverson, Chesham, UK).

Each finished inhaler was prepared to deliver a targeted amount of 90 micrograms of albuterol free base per actuation (measured ex-actuator). The targeted amount was selected to match the manufacturer's reported delivery of 90 micrograms of albuterol (as the free base) from the mouthpiece per actuation for MDIs of Comparative Example 2 (VENTOLIN EVOHALER brand MDI from GlaxoSmithKline plc, Brentford, UK).

### Comparative Example 1

Metered dose inhalers (MDIs) were prepared according to the procedure described in Example 1 with the exception that uncoated 25 microliter 3M retention type valves with PBT stems and EPDM diaphragm seals were used.

### Comparative Example 2

VENTOLIN EVOHALER brand albuterol sulfate MDIs (manufactured by GlaxoSmithKline plc, Brentford, UK) were purchased from a commercial pharmacy. Examination of the VENTOLIN EVOHALER MDIs showed that each inhaler contained a 19 mL aluminum canister with the internal surface having a polytetrafluoroethylene (PTFE) coating, a 63 microliter DF316 valve (AptarGroup Inc., Crystal Lake, IL), and an actuator having a 0.5 mm exit orifice diameter and 1.5 mm jet length. The content of albuterol sulfate was determined to be 1.582 mg/mL in HFA-134a. The product was reported by the manufacturer to deliver 90 micrograms of albuterol (as the free base) from the mouthpiece per actuation.

### Delivered Dose Study - Start of Unit Life Delivered Dose

The start of unit life delivered dose was determined using a standard unit spray collection apparatus (USCA) fitted with a filter. For each determination, an MDI was attached to the USCA using a coupler and actuated a single time. Immediately prior to attachment, the MDI was vigorously shaken. Prior to collection of the test sample, the MDI was primed by actuating four times and before each priming actuation the MDI was vigorously shaken. The flow rate through the equipment was regulated to 28.3 L/minute +/- 0.5 L/minute. The test sample deposited in the USCA was collected by rinsing with 40 microliters of collection solvent. The collection solvent was a 60/40 (volume/volume) solution of acetonitrile/acidified water (0.1% phosphoric acid in water). The recovered samples were then analyzed for sample content using an HPLC assay with reference to a known standard. An Agilent 1100 HPLC instrument (Agilent Technologies, Santa Clara, CA) with a UV detector (210 nm) and a Kinetex Core-shell C-18, 4.6 mm by 150 mm, column (Phenomenex, Torrance, CA) was used. The operating column temperature was 30 °C. A gradient elution method was used with mobile phase A being 0.1% phosphoric acid in water and mobile phase B being 0.1% phosphoric acid in acetonitrile. The gradient elution parameters were 93:7 A:B to 81:19 A:B to 93:7 A:B. The injection volume was 45 microliters and the flow rate was 1.0 mL/minute.

For each example and comparative example, three separate MDIs were individually evaluated according to the study method. The mean (n=3) start of unit life delivered dose of albuterol (determined as the free base) was calculated and the results are reported as micrograms/actuation (mcg/actuation) in Table 1. The percent of measured start of life delivered dose compared to the target albuterol free base delivered dose of 90 micrograms/actuation was calculated and is reported in Table 1.

**Table 1. Albuterol Start of Unit Life Delivered Dose Study (calculated and reported based on the free base form of Albuterol)**

| | Start of Unit Life Delivered Dose of Albuterol (mcg/actuation) | Percent of Albuterol Target Dose |
|---|---|---|
| Example 1 | 86.5 | 96.1% |
| Comparative Example 1 | 76.1 | 84.5% |
| Comparative Example 2 | 89.3 | 99.2% |

### Next Generation Impactor (NGI) Studies

The aerodynamic particle size distribution emitted from each MDI was evaluated using a Next Generation Impactor Instrument (MSP Corporation, Shoreview, MN). For each test, an MDI was attached to the throat component (a standard United States Pharmacopeia (USP) induction port) of the NGI instrument and actuated 6 times into the instrument. Prior to each actuation, the MDI was vigorously shaken. Immediately prior to attachment, the MDI was primed by actuating 4 times. Prior to each priming shot the MDI was vigorously shaken. The flow rate through the instrument during testing was regulated at 30 L/minute. The test sample (albuterol sulfate) deposited on the valve stem, actuator, throat assembly (USP induction port), individual collection cups 1-7 (each collection cup was coated with a formulation of 50% volume/volume glycerol in methanol), micro-orifice collector (MOC), and final filter component was collected by rinsing each individual component with collection solvent. The throat assembly was rinsed with 20 mL of collection solvent and all other components were rinsed with 10 mL of collection solvent. The collection solvent was a 10% solution (volume/volume) of acetonitrile in acidified water (0.1% phosphoric acid in water). The recovered samples were then analyzed for sample content using an HPLC assay with reference to a known standard. An Agilent 1100 HPLC instrument (Agilent Technologies) with a UV detector (210 nm) and a Kinetex Core-shell C-18, 4.6 mm by 150 mm, column (Phenomenex) was used. The operating column temperature was 30°C. A gradient elution method was used with mobile phase A being 0.1% phosphoric acid in water and mobile phase B being 0.1% phosphoric acid in acetonitrile. The gradient elution parameters were 93:7 A:B to 81:19 A:B to 93:7 A:B. The injection volume was 30 microliters and the flow rate was 1.0 mL/minute.

Metered dose inhalers (MDIs) of Example 1 and Comparative Examples 1-2 were evaluated for Total Ex-Valve Content, Total Ex-Actuator Content, FPM, and MMAD of albuterol according to the described NGI Impactor Studies procedure. For each example, three individual MDIs were tested and the results are presented as the mean value. The results are reported in Table 2.

Total Ex-valve content was determined as the sum of the sample content from all twelve analyzed components (valve stem through filter). The Total Ex-valve content was calculated and reported as micrograms/actuation (mcg/actuation) of albuterol free base.

Total Ex-actuator content was determined as the sum of the sample content from the 10 analyzed components positioned after the actuator component (i.e. throat assembly through filter). The Total Ex-actuator content was calculated and reported as micrograms/actuation of albuterol free base.

Fine particle mass (FPM) was calculated using CITDAS (Copley Inhaler Testing Data Analysis Software, from Copley Scientific, Nottingham, UK) as the sum of sample content with a particle size less than 5 micrometers. The FPM was reported as micrograms per actuation of albuterol free base.

The mass median aerodynamic diameter (MMAD) of albuterol particles was calculated and reported using CITDAS (Copley Inhaler Testing Data Analysis Software).

**Table 2. Albuterol Recovered from NGI Studies of MDIs (calculated and reported based on the free base form of Albuterol)**

| | Total Ex-valve Content (mcg/actuation) | Total Ex-Actuator Content (mcg/actuation) | FPM (mcg/actuation) | MMAD (micrometers) |
|---|---|---|---|---|
| Example 1 | 99.3 | 84.9 | 50.4 | 2.5 |
| Comparative Example 1 | 82.5 | 71.3 | 45.0 | 2.5 |
| Comparative Example 2 | 103.5 | 85.5 | 39.2 | 2.4 |

## Claims

1. An inhaler comprising:
a valve having a valve volume of no more than 40 microliters (µL);
a valve coating on at least a portion of the valve, the coating comprising the condensation product of a first layer on the valve and a second layer on the first layer;
the first layer comprising a silane having one or more reactive silane groups, and
the second layer comprising a polyfluoropolyether silane;
a pressurized canister in fluid communication with the valve, the pressurized canister containing a pharmaceutically acceptable inhalable composition that comprises
a propellant; and
albuterol, one or more pharmaceutically acceptable salts of albuterol, or one or more pharmaceutically acceptable hydrates of any of the foregoing,
the albuterol, one or more pharmaceutically acceptable salts of albuterol, or one or more pharmaceutically acceptable hydrates of any of the foregoing being present in a concentration of 4 milligrams/milliliter (mg/mL) or greater, wherein the concentration of albuterol, one or more pharmaceutically acceptable salts of albuterol, or one or more pharmaceutically acceptable hydrates of any of the foregoing is expressed in terms of free albuterol.

2. The inhaler of claim 1, wherein the silane having one or more reactive silane groups comprises a compound of Formula (I)
X₃₋ₘ (R¹)ₘSi - Q - Si(R²)ₖ X₃₋ₖ (I)
wherein
R¹ and R² are independently selected from C₁-C₄ alkyl,
X is a hydrolysable group or a hydroxy group,
m and k are independently 0, 1, or 2, and
Q is a divalent organic linking group,
wherein Q comprises a substituted or unsubstituted C₂ to C₁₂ hydrocarbyl chain, wherein the hydrocarbyl chain is optionally a substituted or unsubstituted C₂ to C₁₂ alkyl chain.

3. The inhaler of any of the preceding claims, wherein the silane having one or more reactive silane groups comprises a mixture of two or more of 1,2-bis(trialkoxysilyl)ethane, 1,6-bis(trialkoxysilyl)hexane, 1,8-bis(trialkoxysilyl)octane, 1,4-bis(trialkoxysilylethyl)benzene, bis(trialkoxysilyl)itaconate, and 4,4'-bis(trialkoxysilyl)-1,1'-diphenyl.

4. The inhaler of any of the preceding claims, wherein the perfluoropolyether silane is a compound of Formula (la)
R*^{f}* [Q¹-[C(R)₂-Si(Y)₃₋ₓ(R^{1a})ₓ]_{y}]_{z} (Ia)
wherein:
R*^{f}* is a monovalent or multivalent polyfluoropolyether moiety;
Q¹ is an organic divalent or trivalent linking group;
each R is independently hydrogen or a C₁₋₄ alkyl group;
each Y is independently a hydrolysable group;
each R^{1a} is independently a C₁₋₈ alkyl or phenyl group;
x is 0 or 1 or 2;
y is 1 or 2; and
z is 1, 2, 3, or 4.

5. The inhaler of claim 4, wherein the compound of Formula (Ia) is a compound of Formula (Ib)
R*^{f}* [Q¹-[C(R)₂-Si(O-)₃₋ₓ(R^{1a})ₓ]_{y}]_{z} (Ib)
wherein:
R*^{f}* is a monovalent or multivalent polyfluoropolyether segment;
Q¹ is an organic divalent or trivalent linking group;
each R is independently hydrogen or a C₁₋₄ alkyl group;
each R^{1a} is independently a C₁₋₈ alkyl or phenyl group;
x is 0 or 1 or 2;
y is 1 or 2; and
z is 1, 2, 3, or 4.

6. The inhaler of any of claims 1 to 3, wherein the perfluoropolyether silane is a compound of Formula (Ic)
R*^{f}* Q¹ᵥ[Q²_{w}-[C(R⁴)₂-Si(X)₃₋ₓ(R⁵)ₓ]_{y}]_{z} (Ic)
wherein:
R*^{f}* is a polyfluoropolyether moiety;
Q¹ is a trivalent linking group;
each Q² is an independently selected organic divalent or trivalent linking group;
each R⁴ is independently hydrogen or a C₁₋₄ alkyl group;
each X is independently a hydrolysable or hydroxyl group;
R⁵ is a C₁₋₈ alkyl or phenyl group;
v and w are independently 0 or 1,
x is 0 or 1 or 2;
y is 1 or 2; and
z is 2, 3, or 4.

7. The inhaler of any of claims 4 to 6, wherein R*^{f}* comprises perfluorinated repeating units selected from the group consisting of -(CₙF₂ₙO)-, -(CF(Z)O)-, -(CF(Z)CₙF₂ₙO)-, - (CₙF₂ₙCF(Z)O)-, -(CF₂CF(Z)O)-, and combinations thereof,
wherein n is an integer from 1 to 6, preferably 1 to 4, more preferably 1 to 3, and even more preferably 1 or 2, and
Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6, preferably wherein Z is a perfluoroalkyl group, an oxygen-containing perfluoroalkyl group, a perfluoroalkoxy group, or an oxygen-substituted perfluoroalkoxy group.

8. The inhaler of any of claims 5 to 7 wherein R*^{f}* is selected from the group consisting of - CF₂O(CF₂O)ₘ(C₂F₄O)ₚCF₂-, -CF(CF₃)O(CF(CF₃)CF₂O)ₚCF(CF₃)-, -O ₂O(C₂F₄O)ₚ CF₂-, - (CF₂)₃O(C₄F₈O)ₚ(CF₂)₃-, -CF(CF₃)-(OCF₂CF(CF₃))ₚO-CₜF₂ₜ-O(CF(CF₃)CF₂O)ₚCF(CF₃)-, wherein t is 2, 3 or 4, m is 1 to 50, and p is 3 to 40.

9. The inhaler of any of the preceding claims, wherein the valve comprises a valve stem and the coating is disposed on at least a portion of the valve stem.

10. The inhaler of any of the preceding claims, wherein the albuterol, one or more pharmaceutically acceptable salts thereof, or one or more pharmaceutically acceptable hydrates of any of the foregoing is albuterol sulfate.

11. The inhaler of any of the preceding claims, wherein the albuterol, one or more pharmaceutically acceptable salts of albuterol, or one or more pharmaceutically acceptable hydrates of any of the foregoing is present in a concentration of 4.5 mg/mL or greater, wherein the concentration of albuterol, one or more pharmaceutically acceptable salts of albuterol, or one or more pharmaceutically acceptable hydrates of any of the foregoing is expressed in terms of free albuterol.

12. The inhaler of any of the preceding claims, wherein the valve volume is no more than 35 µL, optionally no more than 30 µL, further optionally no more than 27.5 µL, or still further optionally about 25 µL.

13. The inhaler of any of the preceding claims further comprising an actuator that, when actuated, releases about 70 micrograms (µg) to about 140 µg, optionally about 80 µg to about 125 µg, further optionally about 90 µg to about 110 µg, still further optionally about 95 µg to 105 µg, or yet still further optionally about 100 µg of albuterol from the inhaler.

14. The inhaler of any of the preceding claims, wherein an excipient is present within the pressurized canister.

15. The inhaler of claim 14, wherein the excipient is ethanol.

## Patentansprüche

1. Ein Inhalator, umfassend:
ein Ventil mit einem Ventilvolumen von nicht mehr als 40 Mikrolitern (µL);
eine Ventilbeschichtung auf mindestens einem Teil des Ventils, wobei die Beschichtung das Kondensationsprodukt einer ersten Schicht auf dem Ventil und einer zweiten Schicht auf der ersten Schicht umfasst;
wobei die erste Schicht ein Silan mit einer oder mehreren reaktiven Silangruppen umfasst und
die zweite Schicht ein Polyfluorpolyether-Silan umfasst;
einen Druckbehälter, der in Fluidverbindung mit dem Ventil steht, wobei der Druckbehälter eine pharmazeutisch verträgliche inhalierbare Zusammensetzung enthält, welche umfasst:
ein Treibmittel; und
Albuterol, ein oder mehrere pharmazeutisch verträgliche Salze von Albuterol oder ein oder mehrere pharmazeutisch verträgliche Hydrate eines der vorgenannten Stoffe,
wobei das Albuterol, ein oder mehrere pharmazeutisch verträgliche Salze von Albuterol oder ein oder mehrere pharmazeutisch verträgliche Hydrate eines der vorgenannten Stoffe in einer Konzentration von 4 Milligramm/Milliliter (mg/ml) oder mehr vorliegen, wobei die Konzentration von Albuterol, einem oder mehreren pharmazeutisch verträglichen Salzen von Albuterol oder einem oder mehreren pharmazeutisch verträglichen Hydraten eines der vorgenannten Stoffe in Form von freiem Albuterol ausgedrückt wird.

2. Der Inhalator gemäß Anspruch 1, wobei das Silan mit einer oder mehreren reaktiven Silangruppen eine Verbindung der Formel (I) umfasst
X₃₋ₘ (R¹)ₘSi - Q - Si(R²)ₖX₃₋ₖ (I)
wobei
R¹ und R² unabhängig ausgewählt sind aus C₁-C₄-Alkyl,
X eine hydrolysierbare Gruppe oder eine Hydroxygruppe ist,
m und k unabhängig für 0, 1 oder 2 stehen und
Q eine zweiwertige organische Verbindungsgruppe ist,
wobei Q eine substituierte oder unsubstituierte C₂- bis C₁₂-Kohlenwasserstoffkette umfasst, wobei die Kohlenwasserstoffkette gegebenenfalls eine substituierte oder unsubstituierte C₂- bis C₁₂-Alkylkette ist.

3. Der Inhalator gemäß einem der vorhergehenden Ansprüche, wobei das Silan mit einer oder mehreren reaktiven Silangruppen ein Gemisch aus zwei oder mehr von 1,2-bis(Trialkoxysilyl)ethan, 1,6-bis(Trialkoxysilyl)hexan, 1,8-bis(Trialkoxysilyl)octan, 1,4-bis(Trialkoxysilylethyl)benzol, bis(Trialkoxysilyl)itaconat und 4,4'-bis(Trialkoxysilyl)-1,1'-diphenyl umfasst.

4. Der Inhalator gemäß einem der vorhergehenden Ansprüche, wobei das Perfluorpolyether-Silan eine Verbindung der Formel (la) ist
R*^{f}*[Q¹-[C(R)₂-Si(Y)₃₋ₓ(R^{1a})ₓ]_{y}]_{z} (Ia)
wobei:
R*^{f}* eine einwertige oder mehrwertige Polyfluorpolyether-Einheit ist;
Q¹ eine zweiwertige oder dreiwertige organische Verbindungsgruppe ist;
jedes R unabhängig für Wasserstoff oder eine C₁₋₄-Alkylgruppe steht;
jedes Y unabhängig für eine hydrolysierbare Gruppe steht;
jedes R^{1a} unabhängig für eine C₁₋₈-Alkyl- oder Phenylgruppe steht;
x für 0 oder 1 oder 2 steht;
y für 1 oder 2 steht; und
z für 1, 2, 3 oder 4 steht.

5. Der Inhalator gemäß Anspruch 4, wobei die Verbindung der Formel (la) eine Verbindung der Formel (Ib) ist
R*^{f}*[Q¹-[C(R)₂-Si(O-)₃₋ₓ(R^{1a})ₓ]_{y}]_{z} (Ib)
wobei:
R*^{f}* ein einwertiges oder mehrwertiges Polyfluorpolyether-Segment ist;
Q¹ eine zweiwertige oder dreiwertige organische Verbindungsgruppe ist;
jedes R unabhängig für Wasserstoff oder eine C₁₋₄-Alkylgruppe steht;
jedes R^{1a} unabhängig für eine C₁₋₈-Alkyl- oder Phenylgruppe steht;
x für 0 oder 1 oder 2 steht;
y für 1 oder 2 steht; und
z für 1, 2, 3 oder 4 steht.

6. Der Inhalator gemäß einem der Ansprüche 1 bis 3, wobei das Perfluorpolyether-Silan eine Verbindung der Formel (Ic) ist
R*^{f}*Q¹ᵥ[Q²_{w}-[C(R⁴)₂-Si(X)₃₋ₓ(R⁵)ₓ]_{y}]_{z} (Ic)
wobei:
R*^{f}* eine Polyfluorpolyether-Einheit ist;
Q¹ eine dreiwertige Verbindungsgruppe ist;
jedes Q² eine unabhängig ausgewählte zweiwertige oder dreiwertige organische Verbindungsgruppe ist;
jedes R⁴ unabhängig für Wasserstoff oder eine C₁₋₄-Alkylgruppe steht;
jedes X unabhängig für eine hydrolysierbare oder Hydroxylgruppe steht;
R⁵ für eine C₁₋₈-Alkyl- oder Phenylgruppe steht;
v und w unabhängig für 0 oder 1 stehen,
x für 0 oder 1 oder 2 steht;
y für 1 oder 2 steht; und
z für 2, 3 oder 4 steht.

7. Der Inhalator gemäß einem der Ansprüche 4 bis 6, wobei R*^{f}* perfluorierte Wiederholungseinheiten umfasst, ausgewählt aus der Gruppe bestehend aus -(CₙF₂ₙO)-, -(CF(Z)O)-, -(CF(Z)CₙF₂ₙO)-, -(CₙF₂ₙCF(Z)O)-, -(CF₂CF(Z)O)- und Kombinationen davon,
wobei n eine ganze Zahl von 1 bis 6, vorzugsweise 1 bis 4, stärker bevorzugt 1 bis 3 und noch stärker bevorzugt 1 oder 2 ist, und
Z eine Perfluoralkylgruppe, eine sauerstoffhaltige Perfluoralkylgruppe, eine Perfluoralkoxygruppe oder eine sauerstoffsubstituierte Perfluoralkoxygruppe ist, von denen jede linear, verzweigt oder cyclisch sein kann und 1 bis 5 Kohlenstoffatome und bis zu 4 Sauerstoffatome aufweisen kann, wenn sie sauerstoffhaltig oder sauerstoffsubstituiert ist, und wobei für Wiederholungseinheiten, welche Z enthalten, die Anzahl der Kohlenstoffatome in der Sequenz höchstens 6 beträgt, wobei Z vorzugsweise eine Perfluoralkylgruppe, eine sauerstoffhaltige Perfluoralkylgruppe, eine Perfluoralkoxygruppe oder eine sauerstoffsubstituierte Perfluoralkoxygruppe ist.

8. Der Inhalator gemäß einem der Ansprüche 5 bis 7, wobei R*^{f}* ausgewählt ist aus der Gruppe bestehend aus -CF₂O(CF₂O)ₘ(C₂F₄O)ₚCF₂-, -CF(CF₃)O(CF(CF₃)CF₂O)ₚCF(CF₃)-, -CF₂O(C₂F₄O)ₚCF₂-, -(CF₂)₃O(C₄F₈O)ₚ(CF₂)₃-, -CF(CF₃)-(OCF₂CF(CF₃))ₚO-CₜF₂ₜ-O(CF(CF₃)CF₂O)ₚCF(CF₃)-, wobei t für 2, 3 oder 4 steht, m für 1 bis 50 steht und p für 3 bis 40 steht.

9. Der Inhalator gemäß einem der vorhergehenden Ansprüche, wobei das Ventil einen Ventilschaft umfasst und die Beschichtung auf mindestens einem Teil des Ventilschafts angeordnet ist.

10. Der Inhalator gemäß einem der vorhergehenden Ansprüche, wobei das Albuterol, ein oder mehrere pharmazeutisch verträgliche Salze davon oder ein oder mehrere pharmazeutisch verträgliche Hydrate eines der vorgenannten Stoffe Albuterolsulfat ist.

11. Der Inhalator gemäß einem der vorhergehenden Ansprüche, wobei das Albuterol, ein oder mehrere pharmazeutisch verträgliche Salze von Albuterol oder ein oder mehrere pharmazeutisch verträgliche Hydrate eines der vorgenannten Stoffe in einer Konzentration von 4,5 mg/ml oder mehr vorliegt, wobei die Konzentration von Albuterol, einem oder mehreren pharmazeutisch verträglichen Salzen von Albuterol oder einem oder mehreren pharmazeutisch verträglichen Hydraten eines der vorgenannten Stoffe in Form von freiem Albuterol angegeben ist.

12. Der Inhalator gemäß einem der vorhergehenden Ansprüche, wobei das Ventilvolumen nicht mehr als 35 ul, gegebenenfalls nicht mehr als 30 µl, weiterhin gegebenenfalls nicht mehr als 27,5 µl oder noch weiter gegebenenfalls etwa 25 µl beträgt.

13. Der Inhalator gemäß einem der vorhergehenden Ansprüche, ferner umfassend einen Aktuator, welcher bei Betätigung etwa 70 Mikrogramm (µg) bis etwa 140 µg, gegebenenfalls etwa 80 µg bis etwa 125 µg, weiter gegebenenfalls etwa 90 µg bis etwa 110 µg, noch weiter gegebenenfalls etwa 95 µg bis 105 µg oder noch weiter gegebenenfalls etwa 100 µg des Albuterols aus dem Inhalator freisetzt.

14. Der Inhalator gemäß einem der vorhergehenden Ansprüche, wobei ein Exzipient in dem Druckbehälter vorhanden ist.

15. Der Inhalator gemäß Anspruch 14, wobei der Exzipient Ethanol ist.

## Revendications

1. Inhalateur comprenant :
une valve ayant un volume de valve non supérieur à 40 microlitres (µl) ;
un revêtement de valve sur au moins une partie de la valve, le revêtement comprenant le produit de condensation d'une première couche sur la valve et d'une deuxième couche sur la première couche ;
la première couche comprenant un silane ayant un ou plusieurs groupes silanes réactifs, et
la deuxième couche comprenant un polyfluoropolyéther-silane ;
une cartouche pressurisée en communication de fluide avec la valve, la cartouche pressurisée contenant une composition inhalable pharmaceutiquement acceptable qui comprend
un propulseur ; et
de l'albutérol, un ou plusieurs sels pharmaceutiquement acceptables d'albutérol, ou un ou plusieurs hydrates pharmaceutiquement acceptables de l'un quelconque des composés précédents,
l'albutérol, le ou les sels pharmaceutiquement acceptables d'albutérol, ou le ou les hydrates pharmaceutiquement acceptables de l'un quelconque des composés précédents, étant présents à une concentration de 4 milligrammes/millilitre (mg/mL) ou plus, dans lequel la concentration d'albutérol, du ou des sels pharmaceutiquement acceptables d'albutérol, ou du ou des hydrates pharmaceutiquement acceptables de l'un quelconque des composés précédents, est exprimée en termes d'albutérol libre.

2. Inhalateur selon la revendication 1, dans lequel le silane ayant un ou plusieurs groupes silanes réactifs comprend un composé de formule (I)
X₃₋ₘ(R¹)ₘSi-Q-Si(R²)ₖX₃₋ₖ (I)
dans laquelle
R¹ et R² sont indépendamment choisis parmi un alkyle en C₁ à C₄,
X est un groupe hydrolysable ou un groupe hydroxy,
m et k valent indépendamment 0, 1 ou 2, et
Q est un groupe de liaison organique divalent,
dans laquelle Q comprend une chaîne hydrocarbyle en C₂ à C₁₂ substituée ou non substituée, dans laquelle la chaîne hydrocarbyle est éventuellement une chaîne alkyle en C₂ à C₁₂ substituée ou non substituée.

3. Inhalateur selon l'une quelconque des revendications précédentes, dans lequel le silane ayant un ou plusieurs groupes silanes réactifs comprend un mélange de deux ou plus parmi le 1,2-bis(trialcoxysilyl)éthane, le 1,6-bis(trialcoxysilyl)hexane, le 1,8-bis(trialcoxysilyl)octane, le 1,4-bis(trialcoxysilyléthyl)benzène, l'itaconate de bis(trialcoxysilyle), et le 4,4'-bis(trialcoxysilyl)-1,1'-diphényle.

4. Inhalateur selon l'une quelconque des revendications précédentes, dans lequel le perfluoropolyéther-silane est un composé de formule (Ia)
R*^{f}*[Q¹-[C(R)₂-Si(Y)₃₋ₓ(R^{1a})ₓ]_{y}]_{z} (Ia)
dans laquelle :
R*^{f}* est un fragment polyfluoropolyéther monovalent ou multivalent ;
Q¹ est un groupe de liaison organique divalent ou trivalent;
chaque R est indépendamment un hydrogène ou un groupe alkyle en C₁ à C₄ ;
chaque Y est indépendamment un groupe hydrolysable ;
chaque R^{1a} est indépendamment un groupe alkyle en C₁ à C₈ ou un phényle ;
x vaut 0 ou 1 ou 2 ;
y vaut 1 ou 2 ; et
z vaut 1, 2, 3 ou 4.

5. Inhalateur selon la revendication 4, dans lequel le composé de formule (Ia) est un composé de formule (Ib)
R*^{f}*[Q¹-[C(R)₂-Si(O-)₃₋ₓ(R^{1a})ₓ]_{y}]_{z} (Ib)
dans laquelle :
R*^{f}* est un segment polyfluoropolyéther monovalent ou multivalent ;
Q¹ est un groupe de liaison organique divalent ou trivalent;
chaque R est indépendamment un hydrogène ou un groupe alkyle en C₁ à C₄ ;
chaque R^{1a} est indépendamment un groupe alkyle en C₁ à C₈ ou un phényle ;
x vaut 0 ou 1 ou 2 ;
y vaut 1 ou 2 ; et
z vaut 1, 2, 3 ou 4.

6. Inhalateur selon l'une quelconque des revendications 1 à 3, dans lequel le perfluoropolyéther-silane est un composé de formule (Ic)
R*^{f}*Q¹ᵥ[Q²_{w}-[C(R⁴)₂-Si(X)₃₋ₓ(R⁵)ₓ]_{y}]_{z} (Ic)
dans laquelle :
R*^{f}* est un fragment polyfluoropolyéther ;
Q¹ est un groupe de liaison trivalent ;
chaque Q² est un groupe de liaison organique divalent ou trivalent indépendamment choisi ;
chaque R⁴ est indépendamment un hydrogène ou un groupe alkyle en C₁ à C₄ ;
chaque X est indépendamment un groupe hydrolysable ou un groupe hydroxyle ;
R⁵ est un groupe alkyle en C₁ à C₈ ou un phényle ;
v et w valent indépendamment 0 ou 1 ;
x vaut 0 ou 1 ou 2 ;
y vaut 1 ou 2 ; et
z vaut 2, 3 ou 4.

7. Inhalateur selon l'une quelconque des revendications 4 à 6, dans lequel R*^{f}* comprend des motifs répétitifs perfluorés choisis dans le groupe constitué par -(CₙF₂ₙO)-, -(CF(Z)O)-, -(CF(Z)CₙF₂ₙO)-, -(CₙF₂ₙCF(Z)O)-, -(CF₂CF(Z)O)-, et leurs combinaisons,
dans lesquels n est un entier de 1 à 6, de préférence de 1 à 4, mieux encore de 1 à 3, et plus préférentiellement 1 ou 2, et
Z est un groupe perfluoroalkyle, un groupe perfluoroalkyle contenant de l'oxygène, un groupe perfluoroalcoxy, ou un groupe perfluoroalcoxy substitué par de l'oxygène, chacun pouvant être linéaire, ramifié ou cyclique, et ayant 1 à 5 atomes de carbone et jusqu'à 4 atomes d'oxygène lorsqu'il contient de l'oxygène ou est substitué par de l'oxygène, et dans lequel, pour les motifs répétitifs incluant Z, le nombre d'atomes de carbone en séquence est d'au plus 6, de préférence dans lequel Z est un groupe perfluoroalkyle, un groupe perfluoroalkyle contenant de l'oxygène, un groupe perfluoroalcoxy, ou un groupe perfluoroalcoxy substitué par de l'oxygène.

8. Inhalateur selon l'une quelconque des revendication 5 à 7, dans lequel R*^{f}* est choisi dans le groupe constitué par -CF₂O(CF₂O)ₘ(C₂F₄O)ₚCF₂-, -CF(CF₃)O(CF(CF₃)CF₂O)ₚCF(CF₃)-, -CF₂O(C₂F₄O)ₚ CF₂-, -(CF₂)₃O(C₄F₈O)ₚ(CF₂)₃-, -CF(CF₃)-(OCF₂CF(CF₃))ₚO-CₜF₂ₜ-O(CF(CF₃)CF₂O)ₚCF(CF₃)-, dans lesquels t vaut 2, 3 ou 4, m vaut de 1 à 50, et p vaut de 3 à 40.

9. Inhalateur selon l'une quelconque des revendications précédentes, dans lequel la valve comprend une tige de valve et le revêtement est disposé sur au moins une partie de la tige de valve.

10. Inhalateur selon l'une quelconque des revendications précédentes, dans lequel l'albutérol, le ou les sels pharmaceutiquement acceptables de celui-ci, ou le ou les hydrates pharmaceutiquement acceptables de l'un quelconque des composés précédents, sont du sulfate d'albutérol.

11. Inhalateur selon l'une quelconque des revendications précédentes, dans lequel l'albutérol, le ou les sels pharmaceutiquement acceptables d'albutérol, ou le ou les hydrates pharmaceutiquement acceptables de l'un quelconque des composés précédents, sont présents à une concentration de 4,5 mg/mL ou plus, dans lequel la concentration d'albutérol, du ou des sels pharmaceutiquement acceptables d'albutérol, ou du ou des hydrates pharmaceutiquement acceptables de l'un quelconque des composés précédents, est exprimée en termes d'albutérol libre.

12. Inhalateur selon l'une quelconque des revendications précédentes, dans lequel le volume de valve est d'au plus 35 µL, éventuellement d'au plus 30 µL, plus éventuellement d'au plus 27,5 µL, ou encore plus éventuellement d'environ 25 µL.

13. Inhalateur selon l'une quelconque des revendications précédentes, comprenant en outre un actionneur qui, lorsqu'il est actionné, libère environ 70 microgrammes (µg) à environ 140 µg, éventuellement environ 80 µg à environ 125 µg, plus éventuellement d'environ 90 µg à environ 110 µg, encore plus éventuellement d'environ 95 µg à 105 µg, ou plus éventuellement encore environ 100 µg d'albutérol à partir de l'inhalateur.

14. Inhalateur selon l'une quelconque des revendications précédentes, dans lequel un excipient est présent à l'intérieur de la cartouche pressurisée.

15. Inhalateur selon la revendication 14, dans lequel l'excipient est de l'éthanol.
